(19) Europäisches Patentamt
European Patent Office
Office européen des brevets

(11) EP 3 708 649 A1

(12) # EUROPEAN PATENT APPLICATION
published in accordance with Art. 153(4) EPC

(43) Date of publication:
**16.09.2020 Bulletin 2020/38**

(21) Application number: **18875062.4**

(22) Date of filing: **21.06.2018**

(51) Int Cl.:
***C12N 1/20*** *(2006.01)*
***A01N 63/02*** *(2006.01)*
***A23L 33/135*** *(2016.01)*
***A61K 8/99*** *(2017.01)*
***A61Q 19/00*** *(2006.01)*
***C12R 1/01*** *(2006.01)*

(86) International application number:
**PCT/KR2018/007045**

(87) International publication number:
**WO 2019/093611 (16.05.2019 Gazette 2019/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.11.2017 KR 20170149048**

(71) Applicant: **Amtixbio Co., Ltd.**
**Seoul 05836 (KR)**

(72) Inventor: **LEE, Jong Seung**
**Seoul 05836 (KR)**

(74) Representative: **Bringer IP**
**1, Place du Président Thomas Wilson**
**31000 Toulouse (FR)**

(54) **NOVELPEDIOCOCCUS PENTOSACEUS**

(57) The present invention relates to a novel lactic acid bacterial strain and a composition containing the same. In addition, the present invention relates to an antibacterial composition, a cosmetic composition, and functional food, which contain the lactic acid bacterial strain and a culture medium thereof.

**FIG.1**

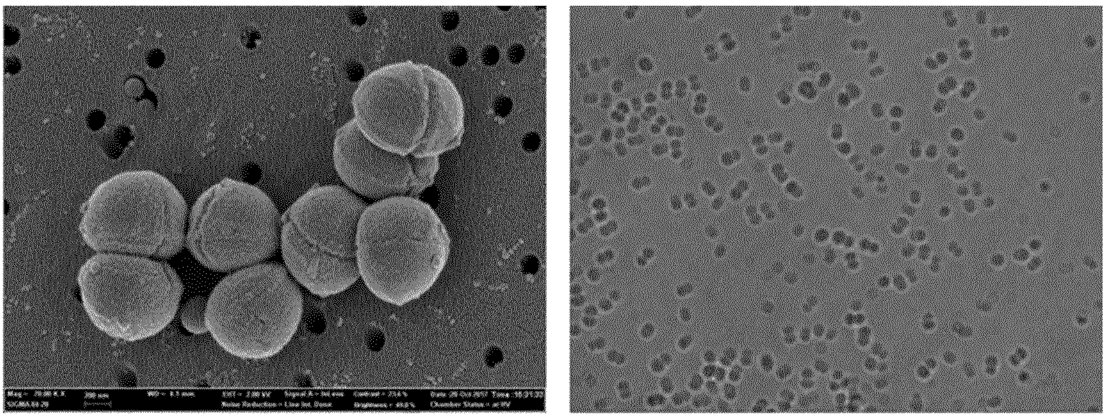

EP 3 708 649 A1

## Description

### TECHNICAL FIELD

The present invention relates to a novel lactic acid bacteria strain and a composition comprising the same. In addition, the present invention relates to an antibacterial composition, a cosmetic composition, and a functional food comprising the lactic acid bacteria strain or culture supernatant thereof.

### BACKGROUND OF THE INVENTION

Lactic acid bacteria decompose carbohydrates, such as, glucose or lactose, and produce lactic acids using the carbohydrates decomposition products. Currently about 300 to 400 lactic acid bacteria are known. Various kinds of lactic acid bacteria are widely distributed in digestive tracts of humans or animals, fermented foods, for example, milk, yogurt, cheese, kimchi, etc., plants and soil, and the like. The representative lactic acid bacteria include *Lactobacillus, Lactococcus, Streptococcus, Leuconostoc, Pediococcus,* and *Bifidobacterium.*

Organic acids, such as, lactic acids and acetic acids produced by lactic acid bacteria exhibit excellent antibacterial activity against harmful bacteria grown in neutral and basic environments, and researches have been conducted to use lactic acid bacteria as preservatives for food, etc. using the antimicrobial properties. In addition, the bacteriocin produced by the lactic acid bacteria is a peptide or a protein-based antibacterial material, and the strains producing these bacteriocins exhibit strong antibacterial activity mainly against the strains which are phylogenetically similar to themselves. Accordingly, attempts have been made to isolate a peptide or a protein having antibacterial activities from lactic acid bacteria, identify characteristics thereof, and develop the same as an antibacterial material or an anti-inflammatory therapeutic agent. In particular, the bacteriocin has an advantage in developing the same as an antibacterial material or an anti-inflammatory therapeutic agent in that the bacteriocin is composed of a peptide or a protein and thus is easily decomposed in a body and thus there is no problem of human toxicity. For example, Nisin has been developed and widely used as a bacteriocin having a broad range of antimicrobial activity.

The tight junction of intestinal epithelial cells is important to maintain homeostasis of the intestinal immune functions. Claudin-4, Zo-1 and Occludin-4 are known as proteins constituting the tight junction, and, in particular, Claudin-1, Zo-1 and Occludin-4 are involved in the tight junctions of skin epithelial cells. It has been found that the function of tight junction is damaged in allergy caused by food, enteritis, autoimmune diseases, and Celiac disease and inflammatory bowel diseases, etc. (Fasano, A., Pathological and therapeutical implications of macromolecule passage through the tight junction. In Tight Junctions, CRC Press, Inc., Boca Raton, Fla. 697-722 (2001)). The tight junction acts as a barrier against the macromolecules entering into a human body. In a healthy state, only a small amount of immunologically active antigens can be introduced into the body through the barrier of the intestinal mucosa. If the tight junction system is damaged by radiation, chemotherapy or toxin or the like, a detrimental immune response leading to harmful autoimmune diseases and food allergy occurs. In the normal intestine, the intestinal immune response is adjusted to maintain the homeostasis of the intestine. The Celiac disease (CD) is a kind of chronic autoimmune disease, and gluten, which is a major protein fraction of wheat, is known as a factor leading to this disease. The digested gluten or derivatives thereof is deamidated by tissue transglutaminase (tTG) after passing through the small intestine epithelial cell wall and induces harmful T cell-mediated immune response while being involved in class II MHC molecules. When the tight junction system is damaged by such as the CD, the paracellular leak (leaky gut) and a response to the environmental antigens occur.

The inflammatory bowel disease (IBD) refers to an undesirable immune response developing in the intestine. Two types of IBD have been known: Crohn's disease and ulcerative colitis (UC). These two types of inflammatory bowel disease show an abnormal profile of T cell-mediated immunity. In patients with Crohn's disease, a strong Th1 response is induced, and in the colon of a UC patient, the Th2 response is raised. In the IBD, the barrier function of the intestine is destroyed. For example, the Crohn's disease is associated with increased permeability of the intestinal barrier (Oshitani, et al. Int. J Mol. Med. 15: 407-10; Ye Arch, et al, Am J Physiol.-Gastro Arch. And Cover Physiol. 290: 496-504, Wilmington, Del. et al. Clin. Exp. Immunol. 142: 275-284).

A tight junction is a barrier that is present in epithelial cells and that secures adjacent cell membrane sides to each other and prevents the flow of water and bodily fluids through the cell's side space. As the stratum corneum is damaged, the concentration of calcium ions is reduced together with the loss of a body fluid existing between the stratum corneum and the granular layer, so that the tight junctions of the upper portion of the granular layer is opened, and the dendrites of the Langerhans cells located just below the tight junctions are raised above the tight functions. The protein antigens with large molecular weight passing through the damaged stratum corneum are collected and recognized by the dendrite of Langerhans cell and transferred to local lymph nodes, and the serine-family proteases of the protein antigens activate PAR-2 present in the keratinocyte membrane. On the other hand, the bacteria that penetrate the stratum corneum activate the toll-like receptor (TLR). The keratinocytes secret various chemokines and cytokines, such

as, TNF-$\alpha$, IL-1 and thymic stromal lymphopoietin (TSLP), etc. to promote the onset of atopic dermatitis and exacerbate the disease. In addition, the expression of the tight junction proteins is significantly reduced in the skin of the atopic dermatitis patient comparing to the normal person. Recently, the Claudin-1 gene (CDN1) mutation has been reported among the atopic dermatitis patients and it is reported that the reduction of Claudin-1 in the tight junctions is one of the reasons for herpes virus infection (De Benedetto A, Rafaels NM, McGirt LY, Ivanov AI, Georas SN, Cheadle C, Berger AE, Zhang K, Vidyasagar S, Yoshida T, Boguniewicz M, Hata T, Schneider LC, Hanifin JM, Gallo RL, Novak N, Weidinger S, Beaty TH, Leung DY, Barnes KC, Beck LA. Tight junction defects in patients with atopic dermatitis. J Allergy Clin Immunol 2011;127:773p786e7.9).

We, inventors, have strived to isolate lactic acid bacteria strains having excellent antibacterial activity as well as antifungal activity, and as a result, confirmed that lactic acid bacteria strains isolated from Korean traditional foods, such as, fermented soybean paste or the like exhibit wide range of antibacterial activities and outstanding antifungal activities. We confirmed that the identified novel lactic acid bacteria belong to the *Pediococcus pentosaceus* and named it as *Pediococcus pentosaceus* AB160011.

## DETAILED DESCRIPTION OF THE INVENTION

### TECHNICAL PROBLEM TO BE SOLVED

The purpose of the present invention is to provide a novel lactic acid bacteria strain having antibacterial and antifungal activities.

In addition, the present invention provides a novel lactic acid bacteria strain which increases the expression of proteins constituting tight junctions.

In addition, the objective of the present invention is to provide a functional food for improving inflammatory bowel disease and immune diseases, comprising the lactic acid bacteria strains of the present invention.

In addition, the objective of the present invention is to provide an antibacterial and antifungal composition comprising the lactic acid bacteria strains above or cell-free culture supernatant of the lactic acid bacteria strains of the present invention.

In addition, the present invention provides a cosmetic composition comprising the lactic acid bacteria strains above or cell-free culture supernatant of the lactic acid bacteria strains of the present invention.

### TECHENICAL SOLUTION

To achieve the purpose, the present invention provides a novel *Pediococcus pentosaceus* AB 160011 strain.

### EFFECT OF THE INVENTION

The *Pediococcus pentosaceus* AB 160011, according to the present invention, has excellent acid resistance and bile resistance, and exhibits antibacterial and antifungal activities. In addition, it has been found that the strain of the present invention and the culture supernatant thereof significantly increase the expression of the proteins constituting the tight junctions of the epithelial cells of the skin and the intestine, and has been shown to have an effect of enhancing skin moisturization according to clinical trials. In addition, the novel lactic acid bacteria strain culture supernatant has anti-inflammatory and antioxidant effects.

### BRIEF DESCRIPTION OF THE DRAWINGS

FIG.1 is a microscopic picture of *Pediococcus pentosaceus* AB 160011 strain of the present invention.

FIG.2 is a phylogenetic tree showing the molecular biological associations of the strain of the present invention.

FIG.3 is a graph showing the DNA sequence of the strain of the present invention.

FIG.4 is a graph showing a growth curve of the strain of the present invention.

FIG.5 is a graph showing pH changes according to the growth time of the strain of the present invention.

FIG.6 shows the results obtained by inducing the expression of Claudin-4 by the strain of the present invention in CaCo2 cells.

FIG.7 shows the results obtained by inducing the expression of Claudin-1 by the culture supernatant of the strain of the present invention in HaCaT cells.

FIG.8 shows selective inhibitory activities against intestinal pathogens and beneficial bacteria of the strain of the present invention.

FIG.9 is a result of measuring the amount of change in Nitric Oxide, an inflammatory response indicator, by the culture supernatant of the strain of the present invention.

FIG.10 is a result of measuring the amount of change in Nitric oxide, an inflammatory response indicator, by the strain of the present invention.

FIG.11 shows the anti-inflammatory effect by the culture supernatant of the strain of the present invention.

FIG.12 shows the antioxidant effects by the culture supernatant of the strain of the present invention.

FIG.13 shows a skin irritation replacement test by the culture supernatant of the strain of the present invention.

FIG.14 shows the skin moisture improvement effects by the culture supernatant of the strain of the present invention.

**BEST MODE**

The present invention will now be described in more detail with reference to the following examples. The following examples are merely illustrative of the present invention, and it is to be understood that the scope of the invention is not limited to the following examples.

**Isolation and identification, and examples of manufacture**

**(1) Isolation of lactic acid bacteria**

Various kinds of lactic acid bacteria were isolated from fermented products including kimchi, soy sauce, and soybean paste prepared in Korea in order to screen lactic acid bacteria strains having antibacterial and antifungal activities, and cultured in a MRS (Difco 288110) solid medium containing 2% agar using a 10-fold dilution method to obtain the colonies. The resulting colonies were classified according to size, color, and characteristics, and then streaked onto another MRS medium to separate a total of 1,785 lactic acid bacteria of the target strains. Each colony of the isolated lactic acid bacteria was streaked again onto MRS agar plates and incubated at 37°C for 24 hours. The isolated lactic acid bacteria were inoculated into the sterilized MRS broth and incubated at 37°C for 24 hours, followed by the addition of 20%(v/v) glycerol and stored in a ultra-low temperature freezer at -80°C.

**(2) Strain Identification**

Morphology analysis

In order to identify the strains, the morphology of the colonies was observed under a microscope after being cultured in MRS plate media. The colonies' morphology is shown in Table 1, and FIG.1 is a microscopic picture of the strain of the present invention.

[Table 1]

| | *Pediococcus pentosaceus* AB160011 |
|---|---|
| phenotype | circular |
| size | 0.8 ~ 1.0 mm |
| color | white |
| opacity | opaque |

(continued)

| | *Pediococcus pentosaceus* AB160011 |
|---|---|
| cell surface morphology | protrusion |
| surface | smoothness |

Identification by 16S rRNA

The novel strain of the present invention was found to have a 16S rRNA sequence as shown in SEQ ID NO: 1. Based on this, the isolated strain was found to have high similarity to *Pediococcus pentosaceus* AJ3053 and named *Pediococcus pentosaceus* AB160011, and deposited with deposition accession no. KCCM11954P at Korean Culture Center of Microorganisms (KCCM) on December 7, 2016.

Identification by DNA sequence analysis

It was confirmed that the novel strain of the present invention has 98.97% homology with *Pediococcus pentosaceus* (ATCC25745 ATP), according to DNA sequence analysis (Gepard, Nucleotide sequences Dot plot) (see FIG.3).

Cell culture property

The novel lactic acid bacteria strains of the present invention were inoculated into a sterile MRS broth medium and incubated at 37°C to observe changes in turbidity (Ultrospec2100pro,

OD625 nm) and acidity (Orion star A211, pH). FIGS.4 and 5 show the growth curve and acidity change of the strain of the present invention.

Cultivation of lactic acid bacteria strains and freeze drying of the cell-free culture supernatant

The composition of the medium for culturing lactic acid bacteria is shown in Table 2, and the manufacturing process is largely classified into as follows: seed culture, main culture, separation of strains and cell-free culture supernatant, and freeze-drying. The seed culture refers to the process of culturing the strains to a volume of 10% of the final culture volume, depending on the size of the target final culture volume. After the seed culture is completed, the obtained seed culture solution including the strains is inoculated into another sterilized culture medium having larger volume and cultured, which is main culture. After the completion of the said main culture, the lactic acid bacteria and the culture supernatant are separated by centrifugation and/or using ceramic filter to obtain cell-free strain culture supernatant. The separated lactic acid bacteria and the cell-free culture supernatant are freeze-dried through a pre-freezing process and processed into a final powder form stored at -20°C for the following use.

[Table 2]

| | Concentration (g/L) |
|---|---|
| Glucose dextrose | 10 |
| $MgSO_4$ | 0.1 |
| Yeast extract | 5 |
| Whey | 2.5 |
| Sodium acetate | 5 |
| Trisodium citrate | 2 |
| $K_2HPO_4$ | 2 |
| $MnSO_4$ | 0.05 |

## BEST MODE FOR INVENTION

### Example

### Example 1: Culture of pathogens

In order to select lactic acid bacteria inhibiting the growth of pathogenic bacteria, the following bacteria were obtained from KCCM and cultured in the solid media presented in Table 3 below. Bacteria: *Staphylococcus aureus* (KCCM11335), *Escherichia coli* (KCCM11234), *Bacillus cereus* (KCCM40935), *Staphylococcus epidermidis* (KCCM40416), *Pseudomonas* (KCCM11321), *Propionibacterium aeruginosa* (KCCM41747), *Salmonella enterica subsp.enterica* (KCCM11806), *Listeria monocytogenes* (KCCM40307). Fungi (including fungi): *Cryptococcus neoformans* (KCCM50785), *Candida albicans* (KCCM11282), *Aspergillus fumigatus* (ATCC Mya-4609), *Aspergillus niger* (KCCM60332), *Fusarium oxysporum* (KCCM44187), *Penicillium chrysogenum* (KCCM60353) and Malassezia furfur (KCCM12679). Next, each colony of the pathogen was inoculated into a nutrient broth medium and incubated for 24 hours at an appropriate temperature (37°C for *E coli, Staphylococcus aureus* and *Bacillus;* 30°C for *Cryptococcus* and *Candida*), followed by the addition of 20% glycerol, and then stored at -80°C cryogenic freezer. In the case of *Aspergillus, Fusarium* and *Penicillium,* the spores were harvested with PBS (phosphate buffer saline) from YPD medium (Sigma-Aldrich) after 7 days incubation at 30°C, followed by the addition of 20% glycerol, and then stored at -80°C, cryogenic freezer.

[Table 3]

| Pathogenic Microorganisms | Nutrient media |
|---|---|
| *E. coli* | LB (Luria Bertani) |
| *Staphylococcus aureus* | TSB (Tryptic Soy Broth) |
| *Bacillus cereus* | NB (Nutrient Broth) |
| *Cryptococcus neoformans* | YM (Yeast Mold) |
| *Candida albicans* | YM (Yeast Mold) |
| *Aspergillus fumigatus* | YPD (Yeast Extract, Peptone, Dextrose) |
| *Aspergillus niger* | PDA (Potato Dextrose Agar) |
| *Fusarium oxysporum* | PDA (Potato Dextrose Agar) |
| *Penicillium chrysogenum* | PDA (Potato Dextrose Agar) |

### Example 2: Detection of antimicrobial and antifungal activities

For the isolated strains, their antimicrobial and antifungal activities were detected. First, each isolated strain stored at -80°C was inoculated onto MRS solid medium and incubated at 37°C for 24 hours, and then the respective colonies were taken and suspended in 500 $\mu\ell$ of PBS to prepare a sample solution for subsequent antibacterial and antifungal activity experiments. Next, media for antimicrobial and antifungal activity tests of the isolation strains were prepared. To this end, the bacteria and fungi cultured and stored in Example 1 were inoculated into a nutrient media suitable for each growth and incubated for 24 hours. Next, the soft agar medium containing MRS (1%, w/w) was sterilized at 121°C for 15 minutes, cooled to 55°C, and each culture medium including bacteria or fungi obtained above was inoculated with 1% (v/v) on the said MRS medium. The soft agar MRS medium inoculated with the bacteria or fungi was then poured into a disposable petri dish and placed at room temperature to prepare an overlay medium for antibacterial or antifungal activity experiments. A sterilized 8 mm paper disk (Adventec, 51020693) was placed on the obtained overlay medium, 50 $\mu\ell$ of the isolated lactic acid bacteria or cell-free culture supernatant of the present invention was added, and incubated at the appropriate growth temperature of each bacteria or fungi for 24 hours. After the culture was completed, the diameter of the clear zone for the pathogens was measured to determine the antibacterial activity and antifungal activity of the isolated lactic acid bacteria. The antibacterial activity and antifungal activity above was indicated according to the diameter of the clear zone as follows: 8 mm or more: +, 13 mm or more: + +, 15 mm or more: + + +, 17 mm or more: + + + +.

**Example 3: Acid resistance and bile resistance of the lactic acid bacteria of the present invention**

The isolated lactic acid bacteria strains were cultured in Lactobacilli MRS broth for more than 16 hours and then the 100 $\mu\ell$ of the bacterial culture was inoculated into 100 $\mu\ell$ of MRS broth with different pH conditions (pH7.0, pH2.5) in a 96 well plate, followed by incubation at 37°C for 24 hours. The survival rate of the lactic acid bacteria was measured by comparing the absorbances of the pH7.0 culture medium and the pH2.5 culture medium at 620 nm using Microplate reader. The cell viability for the acid resistance test was measured in the following manner.

$$\text{Viability (\%)} = OD_{24h,\ pH\ 2.5}/OD_{24h,\ pH\ 7.0} \times 100$$

To confirm bile resistance, the isolated lactic acid bacteria strain was cultured in Lactobacilli MRS broth for more than 16 hours and then 100 $\mu\ell$ of the bacterial culture was inoculated into 100 $\mu\ell$ of MRS broth with two conditions (MRS broth, MRS broth + 0.3% oxgall) in a 96 well plate, followed by incubation at 37°C for 24 hours. The survival rate was measured by comparing the absorbance at 620 nm using Microplate reader. The cell viability for the bile resistance test was measured in the following manner.

$$\text{Viability Measurement (\%)} = OD_{24h,\ MRS\ +\ 0.3\%\ Oxgall}/OD_{24h,\ MRS \times 100}$$

The acid resistance and bile resistance of the novel lactic acid bacteria of the present invention were 86.87% and 99.37%, respectively, and were found to have excellent properties exceeding the general standard of 70%.

**Example 4: Expression of TJ (tight junction) proteins in Caco-2 cells or HaCaT cells by the lactic acid bacteria of the present invention**

The induction of expression of the intestinal TJ constituent proteins was confirmed by using Caco-2 cells (obtained from Korean Cell Line bank), intestinal epithelial cells. As shown in FIG.6, it was confirmed that the cell-free supernatant of the lactic acid bacteria of the present invention effectively induced the expression of ZO-1, Occludin, Claudin-1 and Claudin-4 proteins. Next, HaCaT cells (obtained from Korean Cell Line Bank) were used to confirm the induction of expression of TJ constituent proteins. As a result, as shown in FIG.7, it was confirmed that the said cell-free supernatant of the present invention effectively induced the expression of ZO-1 ATP, Occludin, Claudin-1, and Claudin-4 proteins in the HaCaT cells. Specifically, the freeze-dried powder of the said cell-free culture supernatant was first quantified and dissolved in PBS. To prevent microbial contamination, 0.2 $\mu$m syringe filtration was performed and treated at a concentration suitable for 6 well plate. The cell lysis was then performed using a RIPA/PI buffer, after 24 hours of incubation in a 37°C, CO2 incubator. The protein was quantified via Bradford assay, and then SDS-PAGE was carried out. SDS-PAGE was loaded with 20 $\mu$g per lane, and performed at 150 V for 1 h 30 min running, at 100 V for 1 h transfer (NC membrane) followed by blocking for 1 h (4% SKIM milk). Then, Claudin-1 (1:1000) as a primary antibody was treated for 16 hours and beta-actin (1:1000) was treated for 1 hour. As a secondary antibody, Claudin-1(anti-rabbit, 1:2000) was treated for 1 hour and beta-actin (anti-mouse, 1:2000) was treated for one hour. The band was detected using ECL kit.

**Example 5: Antimicrobial activity test of the lactic acid bacterial culture supernatant of the present invention (MIC, Minimum Inhibitory Concentration)**

The minimum inhibitory concentration (MIC) measurement was performed by a standard test method (Wiegand et al. Nat Protoc. 2008). First, *E. coli, S. aureus* or *B. cereus* was inoculated onto a sterile test solid nutrient medium (MHB, Muller Histone Broth, Difco) and cultured. The *C. neoformans* or *C. albicans* was inoculated on YM nutrient medium (Difco) and *A. fumigatus* was inoculated on YPD nutrient medium (Difco), and incubated at 37°C for 24 hours. The bacteria were then inoculated into sterile liquid medium (MHB) and the fungus was inoculated into RPMI1640 medium, respectively. Next, the cell-free bacterial culture supernatant of the present invention was added to MHB or RPMI1640 medium obtained above and incubated at 37°C for 24 hours to measure the turbidity of the pathogenic microorganisms.

The cell-free culture supernatant solution was prepared by dissolving the freeze-dried lactic acid bacteria culture supernatant in the triple distilled water. The serial 2 x dilution of the supernatant solution said was used to confirm the lowest concentration of the culture supernatant inhibiting the visible growth of pathogens. To confirm the antibacterial and antifungal activities, the test above was repeated three times, and the OD (620 nm) value of the culture supernatant was detected using microplate spectrophotometer (Multiskan FC A28696). As shown in Table 4, the lactic acid bacteria culture supernatant of the present invention exhibited extensive inhibitory activity against Gram-positive and Gram-negative bacteria and fungi.

**Example 6: Antifungal activity test of the lactic acid bacterial culture supernatant of the present invention (MIC)**

Antifungal activity and MIC test were performed according to the standard test method (CLSI document M38-A2, 2008). The culture supernatant prepared in the example above was serially 2 x diluted for this experiment. Each well of each 96 well was inoculated with mold or fungi spores at a concentration of $2 \times 10^3$ spores/mℓ. The plate was incubated at 35°C for 48 hours. The MIC was determined by microscopic observation to the lowest concentration of the cell-free culture supernatant inhibiting the visible growth of the fungi or mold. The experiment was repeated three times to confirm the antifungal activity. Table 4 shows the results.

[Table 4]

| | MIC (mg/mℓ) |
|---|---|
| *Escherichia coli* | 0.7 |
| *Staphylococcus aureus* | 0.9 |
| *Staphylococcus epidermidis* | 1.6 |
| *Propioni bacterium acnes* | 0.8 |
| *Bacillus cereus* | 0.7 |
| *Pseudomonas aeruginosa* | 0.8 |
| *Salmonella* | 0.8 |
| *Listeria* | 0.8 |
| *Cryptococus neoformans* | 25 ~ 50 |
| *Candida albicans* | 25 ~ 50 |
| *Aspergillus niger* | 25 ~ 50 |
| *Fusarium oxysporum* | 25 ~ 50 |
| *Penicillium chrysogenum* | 25 ~ 50 |
| *Malassezia furfur* | 25 ~ 50 |

**Example 7: Thermal stability**

Experiments were conducted to determine whether the antibacterial and antifungal activity against the pathogens by the cell-free culture supernatant of the lactic acid bacteria has thermal stability. The concentrated culture supernatant dissolved solution was prepared in the same manner as in the above example and was divided to two groups of non-heat treatment and heat treatment to determine the MIC showing antibacterial and antifungal activities. For the heat-treated group, the culture supernatant solution was heat-treated in 80°C water bath (Biofree, BF60AC) for 30 minutes or in 121°C autoclave for 15 minutes. The results of the experiments are shown in Table 5.

[Table 5]

| | pathogens | Minimum Inhibitory Concentration (mg/mℓ) | | |
|---|---|---|---|---|
| | | non-heat treatment | 80°C, 30 min. | 121°C, 15 min. |
| G (-) | *E. coli* | 0.7 | 0.7 | 0.7 |
| G (+) | *S. aureus* | 0.9 | 0.9 | 0.9 |
| G (+) | *B. cereus* | 0.7 | 0.7 | 0.7 |
| fungi | *C. neoformans* | 6.25 | 6.25 | 6.25 |
| fungi | *C. albicans* | 25.00 | 25.00 | 25.00 |

**Example 8: pH stability**

Experiments were conducted to determine whether the antibacterial and antifungal activity against pathogens of the lactic acid bacteria culture supernatant has pH stability. The concentrated culture supernatant solution prepared in the same manner as in the above example was treated with non-pH adjusted group or pH adjusted group to measure the MIC showing antibacterial and antifungal activities.

The pH was adjusted using lactic acids and $NaHCO_3$. The results of the experiments are shown in Table 6 below.

[Table 6]

| | pathogens | Minimum Inhibitory Concentration (mg/mℓ) | | |
|---|---|---|---|---|
| | | non-adjusted (pH4.3) | adjusted (pH4.3→7→4.5) | adjusted (pH4.3→7→pH5) |
| G (-) | *E. coli* | 0.7 | 0.7 | 0.7 |
| G (+) | *S. aureus* | 0.9 | 0.9 | 0.9 |
| G (+) | *B. cereus* | 0.7 | 0.7 | 0.7 |
| fungi | *C. neoformans* | 6.25 | 6.25 | 6.25 |
| fungi | *C. albicans* | 25.00 | 25.00 | 25.00 |

**Example 9: Selective inhibition activity of the cell-free culture supernatant of the lactic acid bacteria**

The *Pediococcus pentosaceus* AB 160011 of the present invention, *Lactobacillus brevis* and *E. coli* were cultured until OD600 reached 0.8, respectively. Then, 0.5, 1 and 1.5 mℓ of *Pediococcus pentosaceus* AB 160011, 1 mℓ. of *Lactobacillus brevis* and 1 mℓ. of *E. coli* were used to remove the supernatant using centrifuge, and the precipitated cells were pooled and washed with distilled water (DW). After performing centrifugation again, the supernatant was removed and *Pediococcus pentosaceus AB160011* was pooled in 5 μℓ of DW, and each *Lactobacillus brevis* and *E. coli* were pooled in 1 mℓ of DW. Then, each *Lactobacillus brevis* and *Escherichia coli,* which were pooled in 1 mℓ of DW, were spread evenly in the MRS solid media. Then, *Pediococcus pentosaceus* AB 1600115, which was pooled in 5 μℓ of DW, was spotted on the MRS medium above where *Lactobacillus brevis* or *Escherichia coli,* was spread, at a constant distance. The results were confirmed after 6 ~ 20 hours of incubation and are shown in FIG.8.

**Example 10: Inflammation induction of the cell-free culture supernatant of the lactic acid bacteria**

The amount of Nitric Oxide, an inflammatory response indicator, was measured. First, RAW264.7 cells were seeded in 12 well plates with $1 \times 10^5$ cells per well and were incubated overnight. The 0.2 g of the lyophilized sample of the cell-free culture supernatant of *Pediococcus pentosaceus* AB160011 was dissolved in 1 mℓ of DMEM. The RAW264.7 cells were treated with 0.25, 0.3, 0.35, 0.4, 0.45 and 0.5% (wt/v) of the cell-free culture supernatant dissolved in DMEM above, and incubated for 24 hours. The amount of NO was determined by measuring the absorbance at 540 nm using Nitric Oxide Colorimetric Assay kit (BioVision). As a result, the said culture supernatant of the present invention did not induce an inflammatory response. The results are shown in FIG.9.

RAW R264.7 cells were seeded into 12 well plates at a concentration of $1 \times 10^5$ cells per well. One day after the seeding, each well was treated with *Pediococcus pentosaceus* AB 160011 at each concentration of $5 \times 10^7$, $1 \times 10^8$ and $2 \times 10^8$ CFU/mℓ for 2 hours and then the amount of NO was measured at 540 nm using Nitric Oxide Colorimetric Assay kit (Biovision, Korea). As a result, the lactic acid bacteria of the present invention did not induce an inflammatory response. The results are shown in FIG. 10.

**Example 11: Anti-inflammatory effect of lactic acid bacteria of the present invention**

One day after the seed inoculation of $1 \times 10^5$ cells per well in a 12 well plate of RAW R264.7 cells, $1 \times 10^8$ or $2 \times 10^8$ cfu/mℓ of *Pediococcus pentosaceus* AB 160011 were added to each well, and each well was treated with 1 μg/mℓ of LPS for 24 hours to induce inflammation.

The amount of NO was determined at 540 nm using Nitric Oxide Colorimetric Assay kit (Biovision, Korea). As a result, the lactic acid bacteria of the present invention have been shown to inhibit inflammation reactions. The results are shown in FIG.11.

**Example 12: Measurement of antioxidant effect**

The DPPH (1,1-diphenyl-2-picrylhydrazyl), which is a water-soluble material having a chemically stabilized free radical, has a maximum absorbance between 515 nm and 520 nm, and the radical (DPPH) is extinguished and discolored when the material encounters a material having an antioxidant activity. It is known that a chemically stable 1-diphenyl-2-picylhydryl radical (DPPH) can be used to analyze an antioxidant effects of the extracts containing several antioxidant components, a beverage, an oil, a pure phenol compound, and the like. The cell-free bacterial culture supernatant of the present invention was added to 500 mℓ of 0.2 mM

DPPH, wherein the said culture supernatant was applied at each concentration of 0.1, 0.25, 0.3, 0.35, 0.4, 0.45 and 0.5 (wt/v)% (or 0.1, 0.25, 0.5, 0.75, 1, 1.5 and 2 (wt/v)%) while adjusting the final volume of the mixture to 1 mℓ. The absorbance was measured at 540 nm after the reaction in the dark room for 30 minutes. As a result, as shown in FIG.12, the present lactic acid bacterial culture supernatant was shown to increase the antioxidant effects in a concentration-dependent manner.

**Example 13: Skin irritation replacement test**

A skin irritation test using a SKINETHIC human skin model (EPISKIN) was carried out at Korea Testing & Research Institute (KTR) according to the guidelines for testing toxicology for cosmetics and animal replacement tests (V) / skin irritation test method (Ministry of Food and Drug Safety, MFDS; Guideline-0752-01). The 16 $\mu\ell$ of the test article of the cell-free bacterial culture supernatant was applied to the said human skin model with 1 minute intervals per tissue and exposed for 42 minutes (± 1) in the clean bench. The test material was then removed and washed a total of 25 times with PBS (1 mℓ at one-time). The reversible damage to the said human skin model was evaluated by incubation the treated human skin at 37°C for 42 hours ± 1 hour in a 5% $CO_2$ incubator. Each group of the three groups of the negative control, the positive control, and the test material group was set to have three repetitive samples. The cell viability of the test article was determined to be 86.3 ± 2.9% over 50% of the cell viability baseline and determined to be non-irritating to the skin. The results are shown in FIG.13.

**Example 14: Skin moisturizing improvement effects of a cosmetic essence containing the cell-free lactic acid bacteria culture supernatant of the present invention**

In order to evaluate the effects of improving skin moisturizing of the cosmetic essence product containing the cell-free bacterial supernatant of the present invention, a clinical test was performed at KTR (Korea). The skin moisturizing assay and abnormal response assay were performed for 22 females aged between 29 years and 59 years using Corneometer both before and after the use of the product. The results of skin moisturization have shown a statistically significant increase of the moisture content in the forehead, both cheeks and jaw areas two and four weeks after use compared to before the product was used (see FIG.14). The evaluation of adverse effects by dermatologists did not report any special skin adverse events to the test subjects during the period of use of the test article, nor did any abnormalities be observed in the scientific examination by dermatologists.

**Industrial Applicability**

The present invention confirms that a novel lactic acid bacterial strain isolated from soybean paste, a Korean traditional fermented food, and the cell-free bacterial culture supernatant thereof have excellent antibacterial and antifungal activities, and induce the expression of proteins involved in TJ (tight junctions) of skin and intestinal epithelial cells. In addition, the bacterial strain and the supernatant of present invention have anti-inflammatory and antioxidant effects, and have effects of enhancing skin moisturization according to clinical trials. Therefore, the lactic acid bacterial strain of the present invention could be widely used in a wide range of industrial sectors such as health functional foods, cosmetics and preservatives.

(Deposit No.)

Deposit institution name: Korean Culture Center of Microorganisms (overseas).

Deposit No.: KCCM11954P

Deposit Date: 20161207

<110> AmtixBioCo.、Ltd.

<120> A novel Pediocococcus pentosaceus AB160011 and composition comprising thereof

<130>AMTIX17P01KR

<160> 1

<170>KoPatent In 3.0

<210> 1

<211> 2849

<212>DNA

<213>Pediococcus pentosaceus

<400> 1

tcatggctca ggatgaacgc tggcggcgtg cctaatacat gcaagtcgaa cgaacttccg 60
ttaattgatt atgacgtact tgtactgatt gagattttaa cacgaagtga gtggcgaacg 120
ggtgagtaac acgtgggtaa cctgcccaga agtaggggat aacacctgga aacagatgct 180
aataccgtat aacagagaaa accgcatggt tttcttttaa aagatggctc tgctatcact 240
tctggatgga cccgcggcgt attagctagt tggtgaggta aaggctcacc aaggcagtga 300
tacgtagccg acctgagagg gtaatcggcc acattgggac tgagacacgg cccagactcc 360
tacgggaggc agcagtaggg aatcttccac aatggacgca agtctgatgg agcaacgccg 420
cgtgagtgaa gaagggtttc ggctcgtaaa gctctgttgt taaagaagaa cgtgggtaag 480
agtaactgtt tacccagtga cggtatttaa ccagaaagcc acggctaact acgtgccagc 540
agccgcggta atacgtaggt ggcaagcgtt atccggattt attgggcgta aagcgagcgc 600
aggcggtctt ttaagtctaa tgtgaaagcc ttcggctcaa ccgaagaagt gcattggaaa 660
ctgggagact tgagtgcaga agaggacagt ggaactccat gtgtagcggt gaaatgcgta 720
gatatatgga agaacaccag tggcgaaggc ggctgtctgg tctgcaactg acgctgaggc 780
tcgaaagcat gggtagcgaa caggattaga taccctggta gtccatgccg taaacgatga 840
ttactaagtg ttggagggtt tccgcccttc agtgctgcag ctaacgcatt aagtaatccg 900
cctggggagt acgaccgcaa ggttgaaact caaaagaatt gacgggggcc cgcacaagcg 960
gtggagcatg tggtttaatt cgaagctacg cgaagaacct taccaggtct tgacatcttc 1020
tgacagtcta agagattaga ggttcccttc ggggacagaa tgacaggtgg tgcatggttg 1080
tcgtcagctc gtgtcgtgag atgttgggtt aagtcccgca acgagcgcaa cccttattac 1140
tagttgccag cattaagttg ggcactctag tgagactgcc ggtgacaaac cggaggaagg 1200
tggggacgac gtcaaatcat catgcccctt atgacctggg ctacacacgt gctacaatgg 1260
atggtacaac gagtcgcgag accgcgaggt taagctaatc tcttaaaacc attctcagtt 1320
cggactgtag gctgcaactc gcctacacga agtcggaatc gctagtaatc gcggatcagc 1380
atgccgcggt gaatacgttc ccgggccttg tacacaccgc ccgtcacacc atgagagttt 1440
gtaacaccca aagccggtgg ggtaaccttt taggagctag ccgtctaagt gacagagttg 1500
gagacaaata aaaaaggata atatctccta agatattctc tttaaaaaca agggctttgg 1560
atatcaattt ccgggacgga ccccggaggg gttttttggc tagatcgtgg aggtaaaagg 1620
ctcccccaag gcattggatc agtagcaggc cttgagaggg gtatttgggc cccattggga 1680

atgaaacacg gcccccagatt cctacgggag gcagcagtta tgaatttttc cccaatggac 1740

gcaagtctga tcgacccacc cccgcgtgag tgaagaaggg ttttcggctc gtaaagctct 1800

tgttgttaaa gaagaacgtg ggtaagagta actgtttacc cagtgacggt atttaaccag 1860

aaagccacgg ctaattacgt gccagcagcc gcggtaatac gtaggtggca agcgttatcc 1920

ggatttattg ggcgtaaagc gagcgcaggc ggtcttttaa gtctaatgtg aaagccttcg 1980

gctcaaccga agaagtgcat tggaaactgg gagacttgag tgcagaagag gacagtggaa 2040

ctccatgtgt agcggtgaaa tgcgtagata tatggaagaa caccagtggc gaaggcggct 2100

gtctggtctg caactgacgc tgaggctcga aagcatgggt agcgaacagg attagatacc 2160

ctggtagtcc atgccgtaaa cgatgattac taagtgttgg agggtttccg cccttcagtg 2220

ctgcagctaa cgcattaagt aatccgcctg gggagtacga ccgcaaggtt gaaactcaaa 2280

agaattgacg ggggcccgca caagcggtgg agcatgtggt ttaattcgaa gctacgcgaa 2340

gaaccttacc aggtcttgac atcttctgac agtctaagag attagaggtt cccttcgggg 2400

acagaatgac aggtggtgca tggttgtcgt cagctcgtgt cgtgagatgt tgggttaagt 2460

cccgcaacga gcgcaaccct tattactagt tgccagcatt aagttgggca ctctagtgag 2520

actgccggtg acaaaccgga ggaaggtggg gacgacgtca aatcatcatg ccccttatga 2580

cctgggctac acacgtgcta caatggatgg tacaacgagt cgcgagaccg cgaggttaag 2640

ctaatctctt aaaaccattc tcagttcgga ctgtaggctg caactcgcct acacgaagtc 2700

ggaatcgcta gtaatcgcgg atcagcatgc cgcggtgaat acgttcccgg gccttgtaca 2760

caccgcccgt cacaccatga gagtttgtaa cacccaaagc cggtggggta accttttagg 2820

agctagccgt ctaagtgaca gagttggag 2849

<110> AmtixBio Co., Ltd.

<120> A novel Pediococcus pentosaceus AB160011 and composition comprising thereof

<130> AMTIX17P01KR

<160> 1

<170> KoPatentIn 3.0

<210> 1
<211> 2849
<212> DNA
<213> Pediococcus pentosaceus

<400> 1
tcatggctca ggatgaacgc tggcggcgtg cctaatacat gcaagtcgaa cgaacttccg 60

ttaattgatt atgacgtact tgtactgatt gagattttaa cacgaagtga gtggcgaacg 120

ggtgagtaac acgtgggtaa cctgcccaga agtaggggat aacacctgga aacagatgct 180

aataccgtat aacagagaaa accgcatggt tttcttttaa aagatggctc tgctatcact 240

tctggatgga cccgcggcgt attagctagt tggtgaggta aaggctcacc aaggcagtga 300

tacgtagccg acctgagagg gtaatcggcc acattgggac tgagacacgg cccagactcc 360

tacgggaggc agcagtaggg aatcttccac aatggacgca agtctgatgg agcaacgccg 420

cgtgagtgaa gaagggtttc ggctcgtaaa gctctgttgt taaagaagaa cgtgggtaag 480

agtaactgtt tacccagtga cggtatttaa ccagaaagcc acggctaact acgtgccagc 540

agccgcggta atacgtaggt ggcaagcgtt atccggattt attgggcgta aagcgagcgc 600

aggcggtctt ttaagtctaa tgtgaaagcc ttcggctcaa ccgaagaagt gcattggaaa 660

ctgggagact tgagtgcaga agaggacagt ggaactccat gtgtagcggt gaaatgcgta 720

gatatatgga agaacaccag tggcgaaggc ggctgtctgg tctgcaactg acgctgaggc 780

tcgaaagcat gggtagcgaa caggattaga taccctggta gtccatgccg taaacgatga 840

ttactaagtg ttggagggtt tccgcccttc agtgctgcag ctaacgcatt aagtaatccg 900

cctggggagt acgaccgcaa ggttgaaact caaaagaatt gacgggggcc cgcacaagcg 960

gtggagcatg tggtttaatt cgaagctacg cgaagaacct taccaggtct tgacatcttc 1020

tgacagtcta agagattaga ggttcccttc ggggacagaa tgacaggtgg tgcatggttg 1080

tcgtcagctc gtgtcgtgag atgttgggtt aagtcccgca acgagcgcaa cccttattac 1140

tagttgccag cattaagttg ggcactctag tgagactgcc ggtgacaaac cggaggaagg 1200

tggggacgac gtcaaatcat catgcccctt atgacctggg ctacacacgt gctacaatgg 1260

atggtacaac gagtcgcgag accgcgaggt taagctaatc tcttaaaacc attctcagtt 1320

cggactgtag gctgcaactc gcctacacga agtcggaatc gctagtaatc gcggatcagc 1380

```
atgccgcggt gaatacgttc ccgggccttg tacacaccgc ccgtcacacc atgagagttt      1440
gtaacaccca aagccggtgg ggtaaccttt taggagctag ccgtctaagt gacagagttg      1500
gagacaaata aaaaaggata atatctccta agatattctc tttaaaaaca agggctttgg      1560
atatcaattt ccgggacgga ccccggaggg gttttttggc tagatcgtgg aggtaaaagg      1620
ctcccccaag gcattggatc agtagcaggc cttgagaggg gtatttgggc cccattggga      1680
atgaaacacg gccccagatt cctacgggag gcagcagtta tgaatttttc cccaatggac      1740
gcaagtctga tcgacccacc cccgcgtgag tgaagaaggg ttttcggctc gtaaagctct      1800
tgttgttaaa gaagaacgtg ggtaagagta actgtttacc cagtgacggt atttaaccag      1860
aaagccacgg ctaattacgt gccagcagcc gcggtaatac gtaggtggca agcgttatcc      1920
ggatttattg ggcgtaaagc gagcgcaggc ggtcttttaa gtctaatgtg aaagccttcg      1980
gctcaaccga agaagtgcat tggaaactgg gagacttgag tgcagaagag gacagtggaa      2040
ctccatgtgt agcggtgaaa tgcgtagata tatggaagaa caccagtggc gaaggcggct      2100
gtctggtctg caactgacgc tgaggctcga aagcatgggt agcgaacagg attagatacc      2160
ctggtagtcc atgccgtaaa cgatgattac taagtgttgg agggtttccg cccttcagtg      2220
ctgcagctaa cgcattaagt aatccgcctg gggagtacga ccgcaaggtt gaaactcaaa      2280
agaattgacg ggggcccgca caagcggtgg agcatgtggt ttaattcgaa gctacgcgaa      2340
gaaccttacc aggtcttgac atcttctgac agtctaagag attagaggtt cccttcgggg      2400
acagaatgac aggtggtgca tggttgtcgt cagctcgtgt cgtgagatgt tgggttaagt      2460
cccgcaacga gcgcaaccct tattactagt tgccagcatt aagttgggca ctctagtgag      2520
actgccggtg acaaaccgga ggaaggtggg gacgacgtca aatcatcatg ccccttatga      2580
cctgggctac acacgtgcta caatggatgg tacaacgagt cgcgagaccg cgaggttaag      2640
ctaatctctt aaaaccattc tcagttcgga ctgtaggctg caactcgcct acacgaagtc      2700
ggaatcgcta gtaatcgcgg atcagcatgc cgcggtgaat acgttcccgg gccttgtaca      2760
caccgcccgt cacaccatga gagtttgtaa cacccaaagc cggtggggta accttttagg      2820
agctagccgt ctaagtgaca gagttggag                                        2849
```

## Claims

1. A novel *Pediococcus pentosacus* AB160011 strain (Deposit no.: KCCM11954P).

2. A health functional food for improving inflammatory bowel disease comprising the strain of claim 1.

3. An antimicrobial composition comprising the cell-free culture supernatant of the strain of claim 1.

4. An antifungal composition comprising the cell-free culture supernatant of the strain of claim 1.

**5.** A cosmetic composition comprising the cell-free culture supernatant of the strain of claim 1.

FIG.1

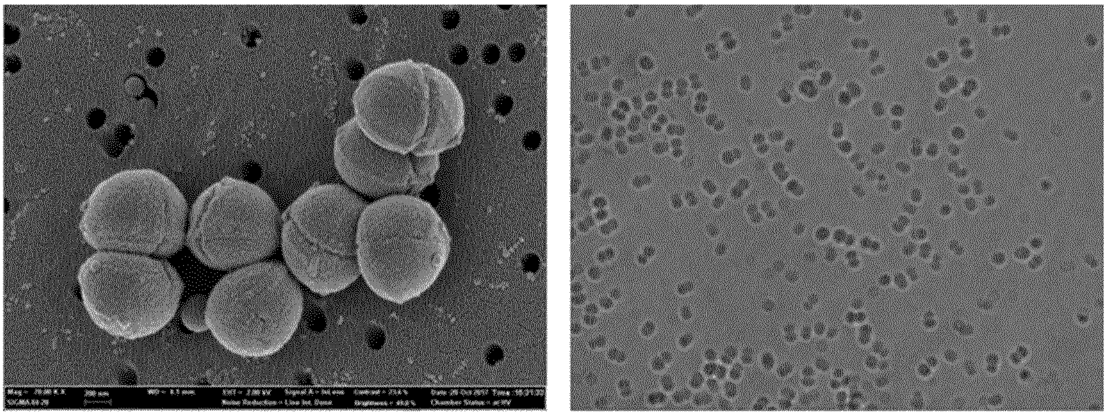

FIG.2

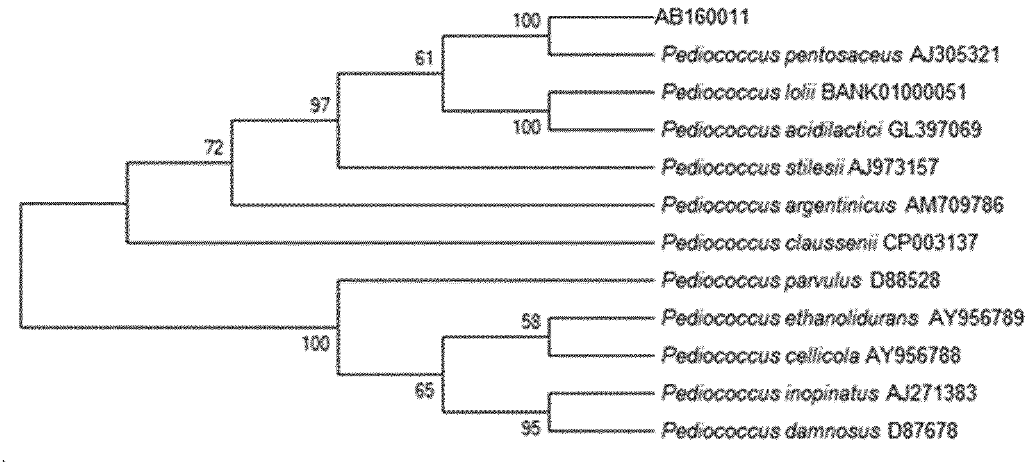


FIG.3

contig.1.cir.fasta

| Contigs | Total length (bp) | A | C | G | T | N | GC content (%) |
|---|---|---|---|---|---|---|---|
| 1 | 1,747,930 | 544,545 | 329,121 | 324,608 | 549,656 | 0 | 37.4 |

GCF_000014505.1_ASM1450v1_genomic.fasta

| Contigs | Total length (bp) | A | C | G | T | N | GC content (%) |
|---|---|---|---|---|---|---|---|
| 1 | 1,832,387 | 573,424 | 342,141 | 342,399 | 574,423 | 0 | 37.36 |

OrthoANIu Results

| Metric | Value |
|---|---|
| OrthoANIu value (%) | 98.97 |
| Genome A length (bp) | 1,747,260 |
| Genome B length (bp) | 1,831,920 |
| Average aligned length (bp) | 1,066,216 |
| Genome A coverage (%) | 61.02 |
| Genome B coverage (%) | 58.2 |

FIG.4

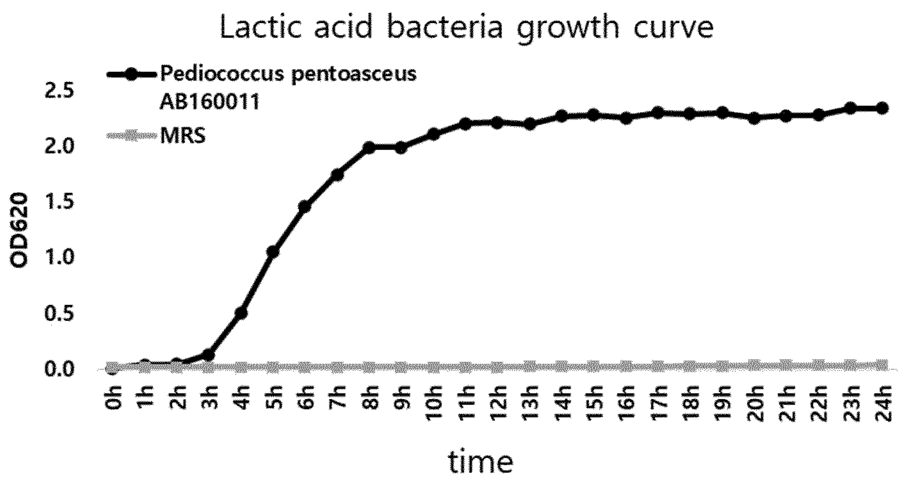


FIG.5

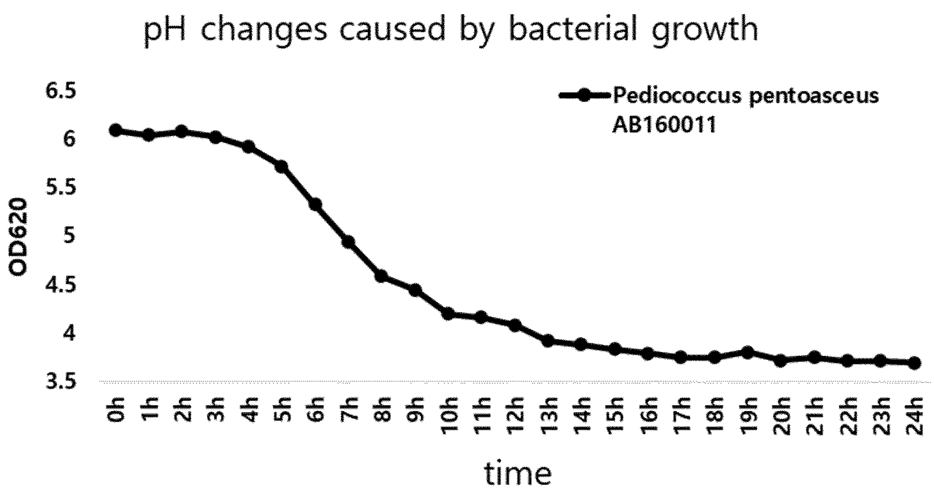


FIG.6

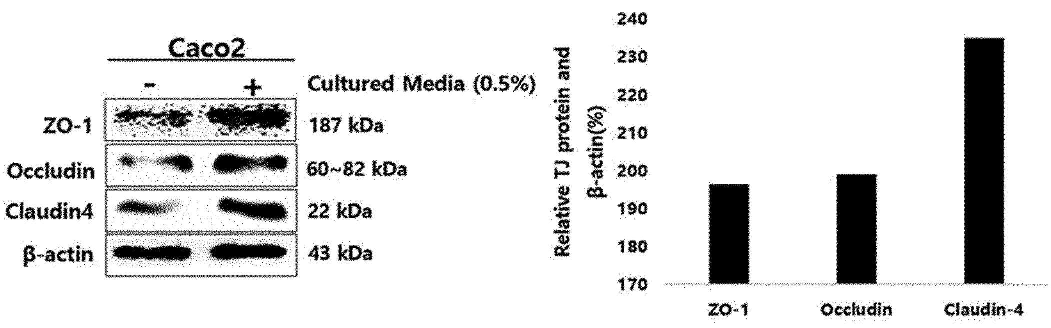

FIG.7

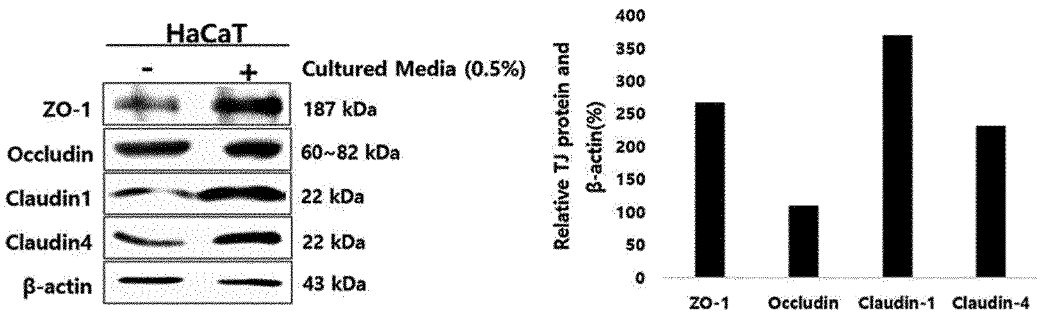


FIG.8

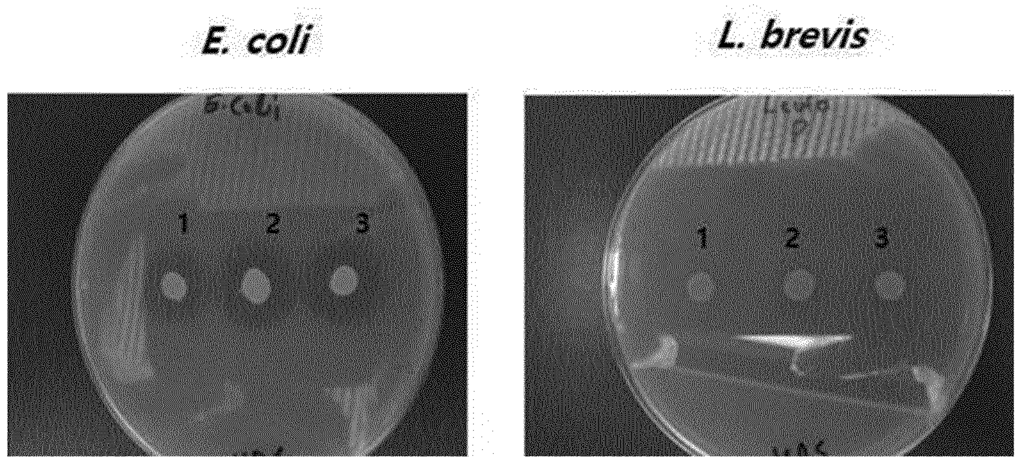


FIG.9

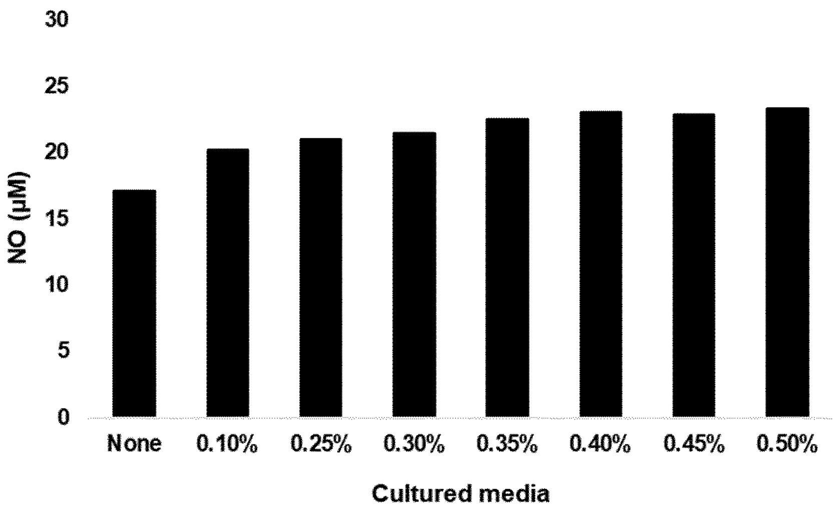

FIG.10

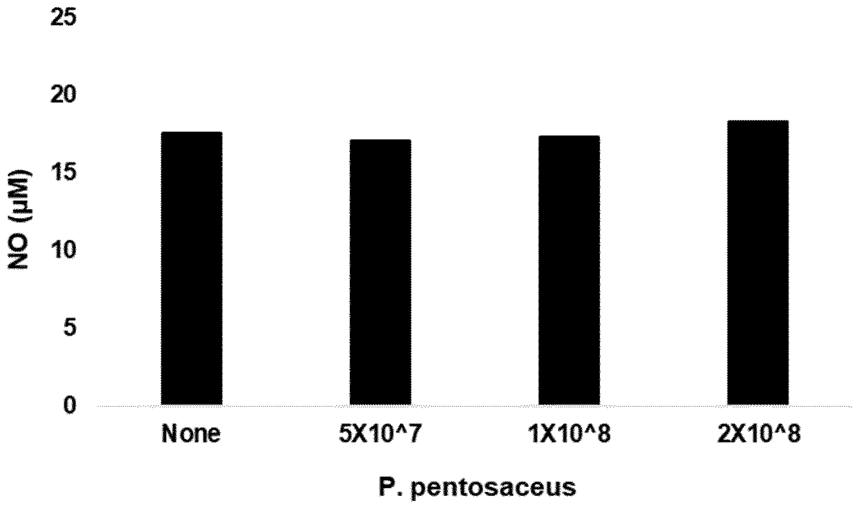
25
20
15
10
5
0
NO (μM)
None
5X10^7
1X10^8
2X10^8
P. pentosaceus

FIG.11

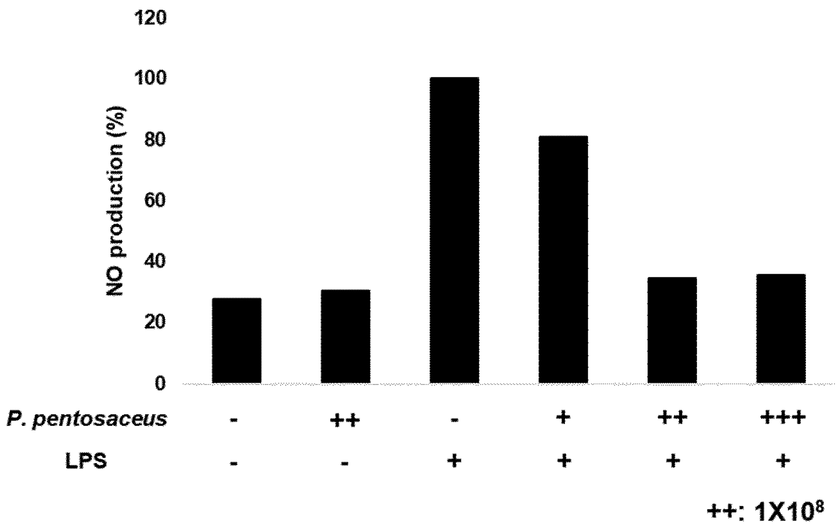
120
100
80
60
40
20
0
NO production (%)
P. pentosaceus
LPS
- ++ - + ++ +++
- - + + + +
++: 1X10^8

FIG.12a

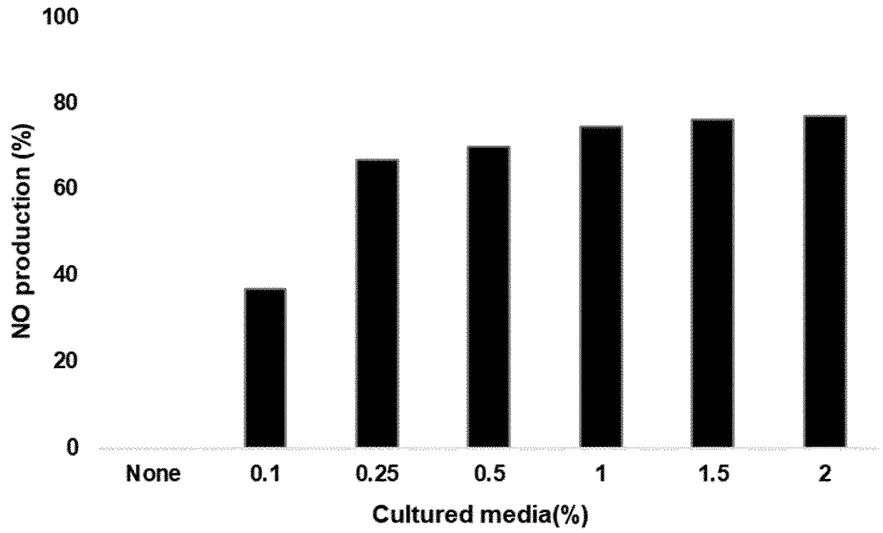


FIG.12b

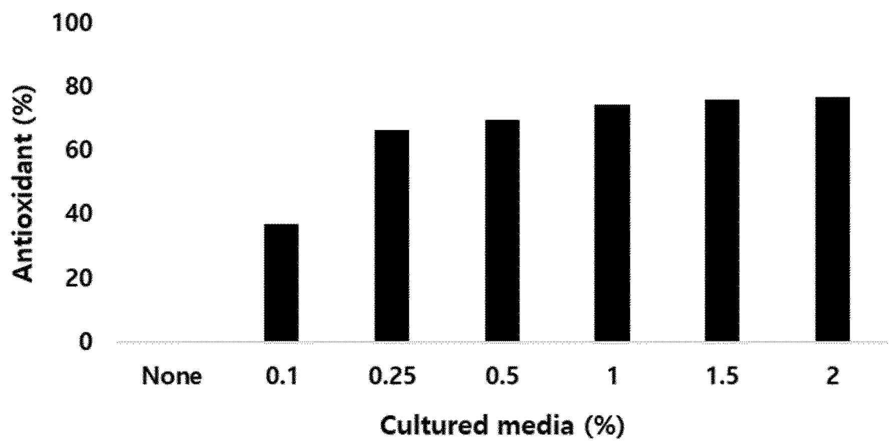

FIG.13

| Group | Substance | Dose (%) | Tissue number | Optical Density (570 nm) | Cell viability (%) |
|---|---|---|---|---|---|
| G1 (NC) | PBS | – | 1101 | 1.992 | 99.2 |
| | | | 1102 | 1.979 | 98.5 |
| | | | 1103 | 2.053 | 102.2 |
| | | | Mean | 2.008 | 100.0 |
| | | | S.D. | 0.040 | 2.0 |
| G2 (PC) | SDS | 5 | 1201 | 0.021 | 1.1 |
| | | | 1202 | 0.020 | 1.0 |
| | | | 1203 | 0.021 | 1.0 |
| | | | Mean | 0.021 | 1.0 |
| | | | S.D. | 0.001 | 0.0 |
| G3 (TS) | water solution of the freeze-dried lactic acid bacteria culture supernatant | 100 | 1301 | 1.760 | 87.7 |
| | | | 1302 | 1.774 | 88.4 |
| | | | 1303 | 1.667 | 83.0 |
| | | | Mean | 1.734 | 86.3 |
| | | | S.D. | 0.059 | 2.9 |

S.D. : Standard deviation, – : Not applicable, NC : Negative control, PC : Positive control, TS : Test substance

FIG.14a

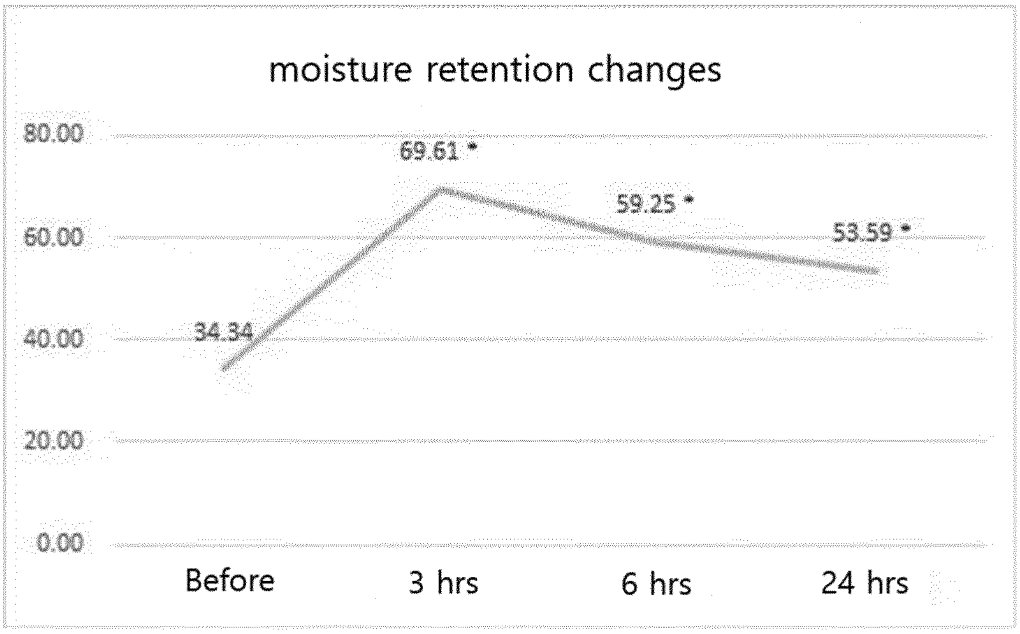

**FIG.14b**

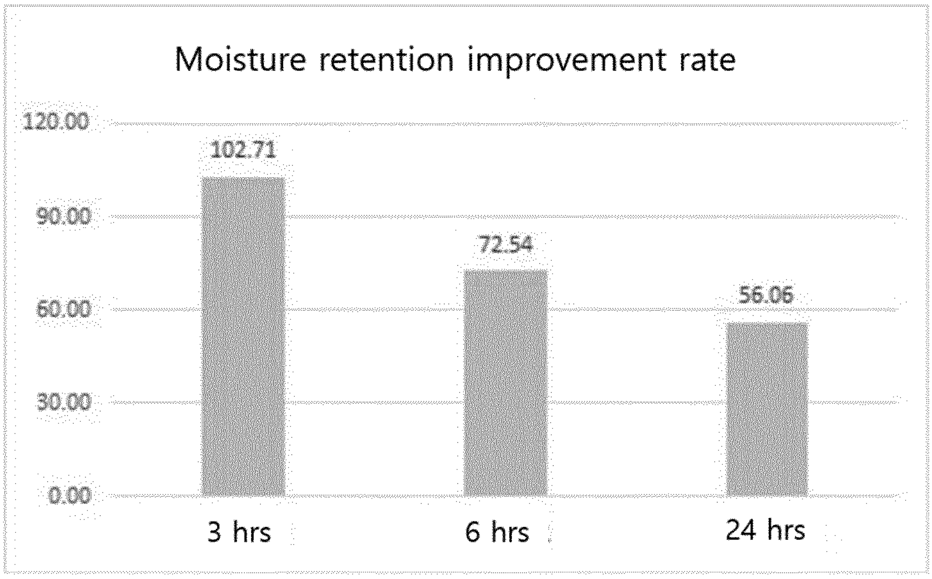
Moisture retention improvement rate
120.00
90.00
60.00
30.00
0.00
102.71
72.54
56.06
3 hrs
6 hrs
24 hrs

## INTERNATIONAL SEARCH REPORT

International application No.
PCT/KR2018/007045

| |
|---|
| A. CLASSIFICATION OF SUBJECT MATTER<br>*C12N 1/20(2006.01)i, A01N 63/02(2006.01)i, A23L 33/135(2016.01)i, A61K 8/99(2006.01)i, A61Q 19/00(2006.01)i, C12R 1/01(2006.01)n*<br>According to International Patent Classification (IPC) or to both national classification and IPC |
| B. FIELDS SEARCHED |
| Minimum documentation searched (classification system followed by classification symbols)<br>C12N 1/20; A23L 11/20; A23L 2/00; A23L 3/3571; A23L 33/00; A61K 35/74; A61K 8/99; A01N 63/02; A23L 33/135; A61Q 19/00; C12R 1/01 |
| Documentation searched other than minimum documentation to the extent that such documents are included in the fields searched<br>Korean Utility models and applications for Utility models: IPC as above<br>Japanese Utility models and applications for Utility models: IPC as above |
| Electronic data base consulted during the international search (name of data base and, where practicable, search terms used)<br>eKOMPASS (KIPO internal) & Keywords: pediococcus pentosaceus, anti-inflammatory, antibacterial, antifungal, antioxidation |

C. DOCUMENTS CONSIDERED TO BE RELEVANT

| Category* | Citation of document, with indication, where appropriate, of the relevant passages | Relevant to claim No. |
|---|---|---|
| X | KR 10-2016-0063025 A (REPUBLIC OF KOREA(MANAGEMENT : RURAL DEVELOPMENT ADMINISTRATION)) 03 June 2016<br>See paragraphs [0002], [0010]-[0015], [0050]-[0060]. | 1-5 |
| X | KR 10-1673450 B1 (MYONGJI UNIVERSITY INDUSTRY AND ACADEMIA COOPERATION FOUNDATION) 09 November 2016<br>See paragraphs [0046], [0071]-[0072], [0095]-[0096]; claims 1, 11. | 1-4 |
| X | KR 10-1650328 B1 (MICROBIAL INSTITUTE FOR FERMENTATION INDUSTRY et al.) 23 August 2016<br>See paragraphs [0060]-[0064]; claim 1. | 1,3,4 |
| X | KR 10-2011-0052809 A (THE DIRECTOR OF CHUNGCHEONGBUK-DO AGRICULTUTAL RESEARCH AND EXTENSION SERVICES et al.) 19 May 2011<br>See paragraphs [0045]-[0059]; claim 1. | 1,3,4 |
| X | KR 10-2016-0039097 A (KOREA FOOD RESEARCH INSTITUTE) 08 April 2016<br>See paragraphs [0094]-[0096]. | 1 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 OCTOBER 2018 (02.10.2018) | **02 OCTOBER 2018 (02.10.2018)** |
| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |

Form PCT/ISA/210 (second sheet) (January 2015)

**INTERNATIONAL SEARCH REPORT**
Information on patent family members

International application No.
**PCT/KR2018/007045**

| Patent document cited in search report | Publication date | Patent family member | Publication date |
|---|---|---|---|
| KR 10-2016-0063025 A | 03/06/2016 | KR 10-1700625 B1 | 13/02/2017 |
| KR 10-1673450 B1 | 09/11/2016 | KR 10-2016-0039015 A | 08/04/2016 |
| KR 10-1650328 B1 | 23/08/2016 | KR 10-2016-0036784 A | 05/04/2016 |
| KR 10-2011-0052809 A | 19/05/2011 | KR 10-1109746 B1 | 13/03/2012 |
| KR 10-2016-0039097 A | 08/04/2016 | NONE | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Pathological and therapeutical implications of macromolecule passage through the tight junction. **FASANO, A.** Tight Junctions. CRC Press, Inc, 2001, 697-722
- **OSHITANI et al.** *Int. J Mol. Med.,* vol. 15, 407-10
- **YE ARCH et al.** *Am J Physiol.-Gastro Arch. And Cover Physiol.,* vol. 290, 496-504
- **WILMINGTON, DEL. et al.** *Clin. Exp. Immunol.,* vol. 142, 275-284
- **DE BENEDETTO A ; RAFAELS NM ; MCGIRT LY ; IVANOV AI ; GEORAS SN ; CHEADLE C ; BERGER AE ; ZHANG K ; VIDYASAGAR S ; YOSHIDA T.** Tight junction defects in patients with atopic dermatitis. *J Allergy Clin Immunol,* 2011, vol. 127, 773-786
- **WIEGAND et al.** *Nat Protoc.,* 2008